# EUROPEAN PATENT SPECIFICATION

(11) **EP 2 756 452 B1**
(45) Date of publication and mention of the grant of the patent: **22.07.2015**
(21) Application number: 12768965.1
(22) Date of filing: 14.09.2012
(51) Int. Cl.: A61B 10/00, A61B 10/02, A61B 5/117, G05B 1/00, A61B 5/15, A61B 5/145, A61B 5/151, A61B 5/157, G06K 9/00

(54) **SIMULTANEOUS ACQUISITION OF BIOMETRIC DATA AND NUCLEIC ACID**
GLEICHZEITIGE AUFNAHME VON BIOMETRISCHEN DATEN UND NUKLEINSÄUREN
ACQUISITION SIMULTANÉE DE DONNÉES BIOMÉTRIQUES ET D'ACIDE NUCLÉIQUE

(30) Priority: 16.09.2011 US 201161535890 P
(43) Date of publication of application: 23.07.2014
(73) Proprietor: Life Technologies Corporation, Carlsbad, California 92008 (US)
(72) Inventor: HARROLD, Michael, Carlsbad, California 92008 (US); HENNESSY, Lori, San Mateo, California 94403 (US); LAGACE, Robert, Carlsbad, California 92008 (US)
(74) Representative: Weber, Birgit
(86) International application number: PCT/US2012/055560
(87) International publication number: WO 2013/040448

(56) References cited:
- WO-A1-99/07282
- WO-A1-2009/021130
- WO-A2-2006/061771
- DE-U1-202005 020 535
- US-A1- 2006 074 280
- US-A1- 2010 191 147
- US-B1- 6 643 531

## Description

### FIELD

The present teachings relate to systems, devices and methods for obtaining biometric data and nucleic acids for use in human identification and forensic science.

### INTRODUCTION

Forensic evidence and biometric data are often used together to identify perpetrators of criminal activities as well as for the identification of missing persons, victims of mass disasters, paternity testing and to exonerate the innocent. The ability to simultaneously collect biometric data such as fingerprints, an iris or retinal scan, an image or photo of an individual can, with a biological sample(s) such as forensic evidence including but not limited to blood, tissue, hair, body fluid or a buccal sample, provide a system for expediting identification, access control, and screening of individuals. Furthermore, maintaining identification of related data points and correlating the data with the respective biological samples can be complicated and susceptible to errors which compromise the chain of custody. Therefore, there remains a need to accurately collect, associate correctly, and process biometric data and biological samples from a single individual in one collection step or workflow.

### SUMMARY

In one aspect, the invention provides a system for collecting a biological sample including at least one nucleic acid and at least one ridge and valley signature of an individual, the system including: a biological collection component including an epidermal puncturing device operationally connected to a presence sensor, where the epidermal puncturing device is configured to be activated to penetrate the epidermis of the individual and to retract, thereby extracting the biological sample; and at least a first imaging component configured to collect the at least one ridge and valley signature, where the at least first imaging component includes i) a platen configured to receive an appendage of the individual, and ii) a presence sensor configured to a) detect when the appendage is placed in proximity to the platen, and b) activate the epidermal puncturing device to extract the biological sample.

In various embodiments, the at least first imaging component is an optical scanner or a capacitance scanner. In some embodiments, the optical scanner is an array of a plurality of light emitting diodes or a multispectral illuminator.

In various embodiments, the presence sensor is a pressure sensor, optical sensor, electronic sensor, capacitive sensor or thermal sensor. In various embodiments, the presence sensor is located a) parallel to the length of a digit, palm, sole, hand or foot; b) perpendicular to the distal end of the digit; or c) resides below the platen, and where the presence sensor activates the epidermal puncturing device to a position above the plane of the platen thereby penetrating the epidermis of the subject.

In various embodiments, the epidermal puncturing device includes an anterior portion configured to penetrate the epidermis, where the anterior portion is selected from the group of a needle, a microneedle, and a lancet; and a posterior portion configured to be operationally connected to the presence sensor. In some embodiments, the anterior portion of the epidermal puncturing device further includes a beveled opening. In some embodiments, the anterior portion of the epidermal puncturing device further includes a lumen. In various embodiments, the anterior portion of the epidermal puncturing device is removable. In some embodiments, the biological sample is conducted for collection to a collection substrate positioned perpendicular to and surrounding the posterior portion of the epidermal puncturing device; a collection substrate positioned within a lumen of the epidermal puncturing device; or a lumen of the epidermal puncturing device. In some embodiments, the system is configured to apply vacuum to the epidermal puncturing device to assist in collecting the biological sample.

In various embodiments, the biological sample is a blood sample, a body fluid sample, or a tissue sample.

In some embodiments, when the epidermis penetrated by the epidermal puncturing device is located on a finger or a hand, then the at least one ridge and valley signature is obtained from at least one finger or the palm. In other embodiments, when the epidermis penetrated by the epidermal puncturing device is located on a toe or a foot, then the at least one ridge and valley signature is obtained from the sole of the foot. In some embodiments, the at least one ridge and valley signature is obtained from at least one finger.

The system may further include a processor configured to transmit the ridge and valley signature of the individual to at least one database including ridge and valley signatures selected from the group consisting of a forensic, criminal, identity, child identity, missing persons, immigration, department of motor vehicles, terrorist, paternity, arrestee, convict database, access control and any combination thereof.

The system may further include an identifier configured to associate the biological sample of the individual with the at least one ridge and valley signature of the individual, wherein the identifier is alphanumeric, graphical, magnetic, or electromagnetic. In some embodiments, the identifier is a barcode.

The system may further include at least a second imaging component for collecting at least a second image of the individual, selected from a facial image, an iris image, a retinal image or an ear image of the individual. In some embodiments, an identifier further associates the at least second image of the individual with the biological sample and the at least one ridge and valley signature of the individual.

In another aspect, the invention provides a method of identifying an individual by collecting a biological sample including at least one nucleic acid sample and at least one ridge and valley signature of a individual, including the steps of: providing an imaging component having a platen surface and a presence sensor where the platen surface is configured to permit an energy wave to penetrate the platen; positioning an appendage of an individual in proximity to the platen and the presence sensor, where when the presence sensor detects the proximity of the appendage then the presence sensor activates an epidermal puncturing device to contact the appendage and to penetrate the epidermis of the appendage; collecting the biological sample to the epidermal puncturing device; and collecting the at least one ridge and valley signature imaged by the energy wave.

In some embodiments the step of collecting the at least one ridge and valley signature is performed electronically. In some embodiments, the steps of collecting the biological sample and collecting the at least one ridge and valley signature are performed at the same time.

In various embodiments, the imaging component is an optical scanner or a capacitance scanner. In various embodiments, the presence sensor detects the proximity of the appendage via pressure, an optical signal, an electronic signal, capacitance or temperature. In various embodiments, the biological sample is a blood sample, a body fluid sample, or a tissue sample.

In various embodiments, when the step of collecting the biological sample is performed by penetrating the epidermis of a finger or a hand, then the at least one ridge and valley signature is obtained from at least one finger or the palm; or when the step of collecting the biological sample is performed by penetrating the epidermis of a toe or a foot, then the at least one ridge and valley signature is obtained from the sole of the foot.

In various embodiments, the epidermal puncturing device includes: an anterior portion configured to penetrate the epidermis, where the anterior portion is selected from the group of a needle, a microneedle, and a lancet; a posterior portion configured to be operationally connected to the presence sensor; and, optionally, where the anterior portion is removable.

The method may include the step of conducting the biological sample for collection to a) a collection substrate positioned perpendicular to and surrounding the posterior portion of the epidermal puncturing device; b) a collection substrate positioned within a lumen of the epidermal puncturing device; or c) a lumen of the epidermal puncturing device.

The method may include the step of applying vacuum to the epidermal puncturing device to collect the biological sample.

The method may additionally include the step of providing an identifier configured to associate the biological sample of the individual with the at least one ridge and valley signature of the individual, wherein the identifier is alphanumeric, graphical, magnetic, or electromagnetic. In some embodiments, the identifier is a barcode.

The method may include the step of transmitting the ridge and valley signature of the individual to at least one database including ridge and valley signatures selected from the group consisting of a forensic, criminal, identity, child identity, missing persons, immigration, department of motor vehicles, terrorist, paternity, arrestee, convict database, access control or any combination thereof.

The method may further include the step of subjecting the biological sample to at least one of a polymerase chain reaction, a DNA sequencing reaction, STR analysis, SNP analysis, or indel analysis. In some embodiments, the at least one nucleic acid of the collected biological sample is not isolated or purified before being subjected to at least one of a polymerase chain reaction, a DNA sequencing reaction, STR analysis, SNP analysis, or indel analysis.

The method may also include the step of collecting at least a second image of the individual, selected from a facial image, an iris image, a retinal image or an ear image of the individual. In some embodiments, an identifier associates the at least second image with the biological sample and the at least one ridge and valley signature of the individual.

In another aspect, the invention provides a device for collecting a biological sample including at least one nucleic acid from an individual, where the device includes: an anterior portion having a lumen, a first end configured to penetrate at least into a dermal layer of the individual, and a second end; a posterior portion configured to be operatively connected to a presence sensor, where the posterior portion optionally has a collection substrate surrounding or inside a lumen of the posterior portion; and further wherein the second end of the anterior portion is connected to the posterior portion. In some embodiments, the first end of the anterior portion is pointed. In various embodiments, the pointed first end further includes a bevel. In some embodiments, the pointed first end further includes at least one or more of a plurality of grooves, at least one threaded groove, at least one barb or serration, and a plurality of micropores. In other embodiments, the collection substrate is selected from filter paper, FTA® paper, Bode™ collection paper, a fibrous material and cotton.

In another aspect, the invention provides a kit comprising the device for collecting a biological sample including at least one nucleic acid from an individual, where the device includes: an anterior portion having a lumen, a first end configured to penetrate at least into a dermal layer of the individual, and a second end; a posterior portion configured to be operatively connected to a presence sensor, where the posterior portion optionally has a collection substrate surrounding or inside a lumen of the posterior portion; and further wherein the second end of the anterior portion is connected to the posterior portion, and optionally, instructions for use. In some embodiments, the first end of the anterior portion of the device of the kit is pointed. In various embodiments, the pointed first end further includes a bevel. In some embodiments, the pointed first end further includes at least one or more of a plurality of grooves, at least one threaded groove, at least one barb or serration, and a plurality of micropores. In other embodiments, the collection substrate is selected from filter paper, FTA® paper, Bode™ collection paper, a fibrous material and cotton. In some embodiments, the kit may include reagents for the acquisition of the biological sample. The kit may further include reagents for archiving, shipment, or further processing of the biological sample.

In another aspect of the invention, there is disclosed an apparatus for collecting at least one nucleic acid sample and at least one ridge and valley signature of an individual, the apparatus having: a biological collection component wherein the device comprises an epidermal puncturing device (EPD) capable of penetrating the epidermis of the individual to extract a biological sample, wherein the biological sample comprises nucleic acid; and an at least first imaging component for collecting the at least one ridge and valley signature.

In accordance with another embodiment disclosed is a method for collecting at least one nucleic acid sample and at least one ridge and valley signature, the method comprising:

providing a platen surface wherein the platen surface allows a light source to penetrate the platen; and in a single workflow performing at least the following: positioning a member of an individual over and in direct contact with the platen and a pressure sensor, wherein the pressure sensor is perpendicular to the horizontal plane comprising the platen, collecting the nucleic acid sample through pressure on the pressure sensor, and collecting the ridge and valley structure through pressure on the platen, wherein the structure is collected from an imaging device.

In the following description, certain aspects and embodiments will become evident. It should be understood that a given embodiment need not have all aspects and features described herein. It should be understood that these aspects and embodiments are merely exemplary and explanatory and are not restrictive of the invention.

There still exists a need for improved apparatus and methods for collecting finger or toe, hand palm or foot sole printing and biological sample data for purposes of identifying and confirming the identity of a human individual.

### BRIEF DESCRIPTION OF THE DRAWINGS

**FIGS. 1A - 1B** illustrate a system for the simultaneous collection of the ridge and valley signature of a digit and a biological sample from an individual shown in **FIG. 1C** and possible sampling areas in **FIG. 1** **D,** in accordance with various embodiments.
**FIG. 2** illustrates potential sampling regions of the palmar and side surface of a hand for obtaining a biological sample, in accordance with various embodiments.
**FIG. 3** is an illustration of a cross-section of skin layers of an individual.
**FIG. 4** contains illustrations of various microneedle configurations, in accordance with various embodiments.
**FIG. 5** illustrates a holder, casing, collection substrate and needle for collection of a biological sample, in accordance with various embodiments.
**FIG. 6A** is a magnified view of a presence sensor activating plate assembly with an epidermal puncturing device.
**FIG. 6B** illustrates two embodiments of epidermal puncturing devices.
**FIG. 7** illustrates a system for the simultaneous collection of a finger or palm print, nucleic acid and an image or biometric scan from an individual, in accordance with various embodiments.
**FIG. 8** illustrates an electropherogram of an STR profile of an individual using nucleic acid collected from a surface of a needle tip of the epidermal puncturing device.
**FIG. 9** illustrates an electropherogram of an STR profile of an individual using nucleic acid collected from the collection substrate.

### DETAILED DESCRIPTION

As used herein, "membrane," "partition," "layer," and "film" are interchangeable and not intended to be limiting.

As used herein, "DNA" and "nucleic acid" are used interchangeably.

As used herein "oligonucleotide" and "polynucleotide" are interchangeable and generally refers to a polymer of nucleotide subunits having a fragment size of about or less than 200 base pairs.

As used herein, "collection substrate" refers to a material capable of absorbing particles or liquid in it.

As used herein, "biological sample" refers to a component originating from either within or on the body of an individual.

As used herein, "blood sample" refers to both the non-cellular components of blood, including but not limited to the serum or plasma, and the cellular components, which include but are not limited to red blood cells, white blood cells or platelets.

As used herein, "body fluid" refers to liquids originating within the body of an individual.

As used herein, "casing" refers to the covering surrounding or holding a needle.

As used herein, "DNA sequencing" refers to the determination of the sequential identity of nucleotides in a molecule of DNA.

As used herein, "epidermal puncturing device" or "EPD" refers to a device for puncturing and penetrating the epidermis layer of skin for the purpose of extracting a biological sample.

As used herein, "filter paper" refers to a semi-permeable paper.

As used herein, "identifier" refers to a label capable of use in cataloging/correlating like-labeled data or data from a single source.

As used herein, "image capturing device" refers to a type of camera or scanning device.

As used herein, "imaging component" refers to an apparatus capable of performing at least one of capturing, developing, storing, retrieving and transmitting an image.

As used herein, "Indel" refers to an insertion or deletion of a segment of nucleic acid, usually DNA, within a nucleic acid sequence.

As used herein, "isolated" refers to separation of nucleic acid from either or both naturally occurring materials or environmental chemicals/substances.

As used herein, "lancet" refers to a pricking needle or double-edged blade.

As used herein, "light emitting diodes" refers to LEDs, a semiconductor light source.

As used herein, "microneedle" refers to a needle with a diameter generally less than about 1 mm and penetration depth generally less than about 3 mm.

As used herein, "multispectral illuminator" refers to a plurality of frequencies/wavelengths across the electromagnetic spectrum used to capture image data.

As used herein, "needle" refers to a hollow cylinder, optionally with a sharp point at one end. The needle may further comprise a beveled area in relation to the sharp point or collection substrate posterior to the pointed end, either located within the lumen or attached to the exterior of the cylinder.

As used herein, "optically collecting" refers to obtaining data, such as a ridge and valley signature or an image through illuminating the data or image and capturing the result.

As used herein, "organ sample" refers to a grouping, by function, of multiple tissues.

As used herein, "digit" refers to one of several most distal parts of a limb, and includes a finger or toe.

As used herein, "photographic apparatus" refers to an LED camera, a digital camera, a still camera, a video camera and a virtual camera.

As used herein, "polymerase chain reaction" or PCR is a an amplification of nucleic acid consisting of an initial denaturation step which separates the strands of a double stranded nucleic acid sample, followed by repetition of (i) an annealing step, which allows amplification primers to anneal specifically to positions flanking a target sequence; (ii) an extension step which extends the primers in a 5' to 3' direction thereby forming an amplicon polynucleotide complementary to the target sequence, and (iii) a denaturation step which causes the separation of the amplicon from the target sequence (Mullis et al., eds, The Polymerase Chain Reaction, BirkHauser, Boston, Mass. (1994)). Each of the above steps may be conducted at a different temperature, preferably using an automated thermocycler (Applied Biosystems LLC, a division of Life Technologies Corporation, Foster City, CA.). If desired, RNA samples can be converted to DNA/RNA heteroduplexes or to duplex cDNA by methods known to one of skill in the art. The PCR method also includes reverse transcriptase-PCR and other reactions that follow principles of PCR.

As used herein, "purified" refers to a nucleic acid sample almost totally absent of other naturally occurring or environmentally occurring materials.

As used herein, "SNP analysis" refers to the evaluation of the presence or absence of a single nucleotide polymorphism (SNP) marker following amplification of the locus containing the SNP marker

As used herein, "STR analysis" refers to the evaluation of the alleles of a short tandem repeat (STR) marker following amplification of the locus containing the STR marker.

As used herein, "tissue sample" refers to a biological sample having a mixture of at least two cell types that carry out a specific function.

As used herein, "topological impression" refers to the ridge and valley topography of a finger, palm, toe or foot.

Reference will now be made to various embodiments, examples of which are illustrated in the accompanying drawings.

The present teachings relate to systems, devices, kits, and methods for collecting at least one nucleic acid sample and at least one ridge and valley signature from an individual. A system according to the invention can find use in, but is not limited to, forensic, criminal, identity, child identity, missing persons, immigration, department of motor vehicles, terrorist, paternity, arrestee, access control or convict database applications. Furthermore, the ability to rapidly correlate the ridge and valley signature of an individual, such as a finger or toe print, palm or sole print with a nucleic acid profile, such as a DNA fingerprint, can provide rapid screening and identification of persons of nefarious intent, suspected of having conducted illegal activities, suspects in criminal investigations and persons of interest and can prevent access of such persons to situations where they might harm others, such as an airline flight, entrance into a high security access area or other sites where access is restricted or poses a security risk. Rapid correlation can also aid in identification and resolution of missing person investigations. In other applications, rapid correlation of nucleic acid profiles and a valley and ridge signature can be used in assisting proper familial identification in immigration and political asylum investigations.

**The system.** A system for collecting at least one nucleic acid sample and at least one ridge and valley signature of an individual includes a biological collection component, where the biological collection component comprises an epidermal puncturing device (EPD) operationally connected to a presence sensor and configured to be activated to penetrate the epidermis of the individual and to retract, thereby extracting a biological sample comprising nucleic acid; and at least a first imaging component configured to collect the at least one ridge and valley signature, where the at least first imaging component comprises a platen configured to receive an appendage of the individual, and a presence sensor. The presence sensor of the system is configured a) to detect when the appendage is in proximity to the platen, and b) to activate the EPD to extract the biological sample. The presence sensor may also trigger the collection of the valley and ridge signature. In other embodiments, a signal from another portion of the system may trigger collection of the valley and ridge signature when the presence sensor detects the presence of an appendage on the platen. The system may collect simultaneously at least one ridge and valley signature of an individual and a biological sample comprising nucleic acid or may collect in succession the signature and biological sample or, visa versa, the biological sample and the signature with an at least first imaging component. The ridge refers to a friction ridge, the raised part of the epidermal layer of the skin of the fingers, toes, palm of the hand or sole of the foot and the valley being the depression in the epidermis between two adjacent ridges The ridges and valleys are commonly referred to as fingerprints, palm prints, toe prints or footprints depending on the origin of the ridge and valley signature.

**At least a first imaging component.** In various embodiments, the system includes at least a first imaging component configured to obtain a ridge and valley signature of the individual. The imaging component may employ energy waves, such as light as described above, or other energy waves such as electromagnetic waves, capacitance, infra-red or sonic, e.g., ultrasound based components to provide an image of the ridge and valley signature. When the term imaging component is used in the context of capturing ridge and valley signatures, this includes any component that captures a digital or analog electronic representation of ridge and valley signatures.

Ridge and valley signatures may also be obtained using a touchless three-dimensional ridge and valley scanner using a digital processing means. (Wang, Yongchang; Q. Hao, A. Fatehpuria, D. L. Lau and L. G. Hassebrook (2009). "Data Acquisition and Quality Analysis of 3-Dimensional Fingerprints". Florida: IEEE conference on Biometrics, Identity and Security. http://vis.uky.edu/~realtime3d/Doc/3D_Fingerprint_Quality.pdf. Retrieved March 2010. Wang, Yongchang; D. L. Lau and L. G. Hassebrook (2010). "Fit-sphere unwrapping and performance analysis of 3D Fingerprints". Applied Optics. pp. 592-600.

In one embodiment of the present teachings, the system may include at least a first optical imaging component. In other embodiments, the system can include at least a first solid-state ridge and valley signature reader. The optical imaging component has an illuminating means for optically collecting the ridge and valley signature using an optical scanner as is known to one of skill in the art. The optical scanner can be an array of a plurality of light emitting diodes or a multispectral illuminator. In an optical scanner a beam of light passes through the topological impression made by the individual upon exposure to the source of illumination wherein the individual places the finger, hand, palm, toe, sole or foot against a surface of the at least first imaging component.

Any suitable instrumentation may be used to acquire the image of an appendage according to the methods described herein. Some instruments and techniques include but are not limited to those disclosed in US4537484, US6175407, US6665427, US8014581, US8036431, US5177353, US6282303, US 6188781,US6741729, US6122394, US6826000, US6496630, US6628813, US6983062, US7162060, US7164440, US7657067, US8073209, US7190817, US7558410, US7565541, US7995808, US7899217, US7890158, US7835554, US7831072, US7819311, US7804984, US7801339, US7801338, US7751594, US7735729, US7668350, US7627151, US7620212, US7613504, US7545963, US7539330, US7508965, US7460696, US7440597, US7394919, US7386152, US7347365, US7263213, US7203345, US7147153, US6816605, US6628809, US6560352, US20110235872, US20110211055, US20110165911, US20110163163, US20110085708, US20100246902, US20100067748, US20090245591, US20090148005, US20090092290, US20090080709, US20090046903, US20080304712, US20080298649, US20080297788, US20080232653, US20080192988, US20080025580, US20080025579, US20070116331, US20070030475, US20060274921, US20060244947, US20060210120, US20060202028, US20060110015, US20060062438, US20060002598, US20060002597, US20050271258, US20050265586, US20050265585, US20050205667, US20050185847, US20050007582, US20040240712, US20040047493, US20030223621, US20030078504, US20020183624, or US20020009213.

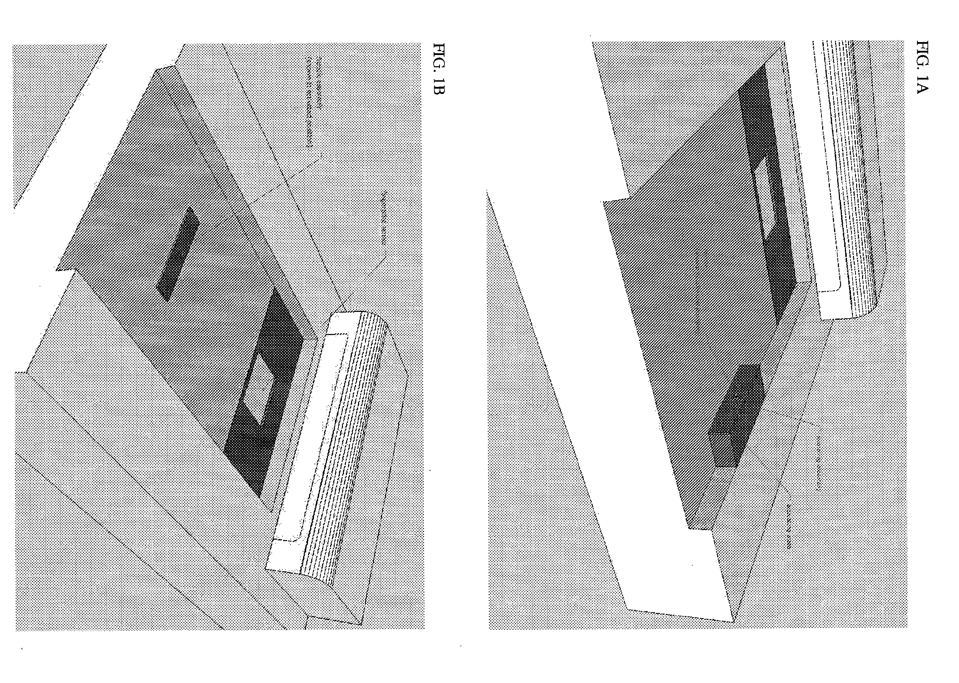

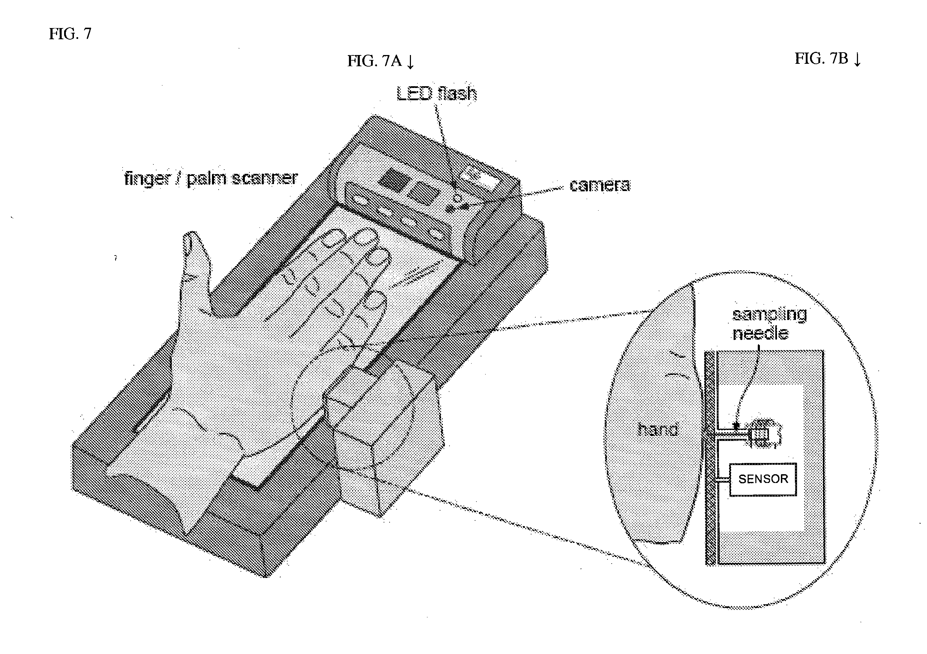

In various embodiments, the at least first imaging component includes a platen configured to receive an appendage of the individual for imaging. The platen may have a planar surface permitting the appendage to be located in proximity to the platen for accurate imaging (as shown in **FIGS. 1A-1D** and **FIG. 7**). In some embodiments, the platen may be interrupted by an opening through which the retractable epidermal puncturing device (EPD) is activated for sample collection and thereafter retracts to a position below the planar surface of the platen (**FIG. 1B**).

The platen is made of material that permits the passage of the energy wave through its upper surface to create a valley and ridge image which is collected by the imaging component. In some embodiments, the platen transmits electrical waves to create a valley and ridge signature via capacitance, which can be sensed by the imaging component and converted into an analog or digital valley and fingerprint signature. In other embodiments, the platen permits the passage of light waves to illuminate a valley and ridge signature via optical imaging, which can be sensed by the imaging component and converted into an analog or digital valley and ridge signature. Alternatively, the platen may permit the passage of infra-red, ultrasound or other energy waves to illuminate a valley and ridge signature of an appendage, including but not limited to a digit, hand or foot of an individual and capture the image in an analog or digital format. The platen surface may be transparent to facilitate the passage of optical or other energy waveforms.

The at least first imaging component of the system includes a presence sensor configured to detect when an appendage of an individual is placed in proximity to the platen. The presence sensor may be a mechanical, optical, capacitive, electromagnetic, infra-red, thermal, or chemical sensor. It may include spring loaded mechanisms, electronic mechanisms or any suitable technology that may detect the presence of the appendage against the platen. The presence sensor is connected operationally, via mechanical, electronic, magnetic, or other functional connection with a retractable epidermal puncturing device (EPD). Upon sensing a preselected proximity of an appendage positioned appropriately on the platen, the presence sensor is configured to activate the EPD to move from an inactive position to an active position. In its active position, the EPD is positioned and configured to penetrate the epidermis of the test subject. In some of the embodiments, the presence sensor is an electronic sensor. In other embodiments, the presence sensor is an optical sensor. In further embodiments, the presence sensor is a pressure sensor. In some other embodiments, the presence sensor is a capacitive sensor. In yet other embodiments, the presence sensor is a thermal sensor. Any type of presence sensor may be used in combination with any suitable imaging component, including not but not limited to an optical or capacitive scanner, and may further be used with any suitable embodiment of the EPD.

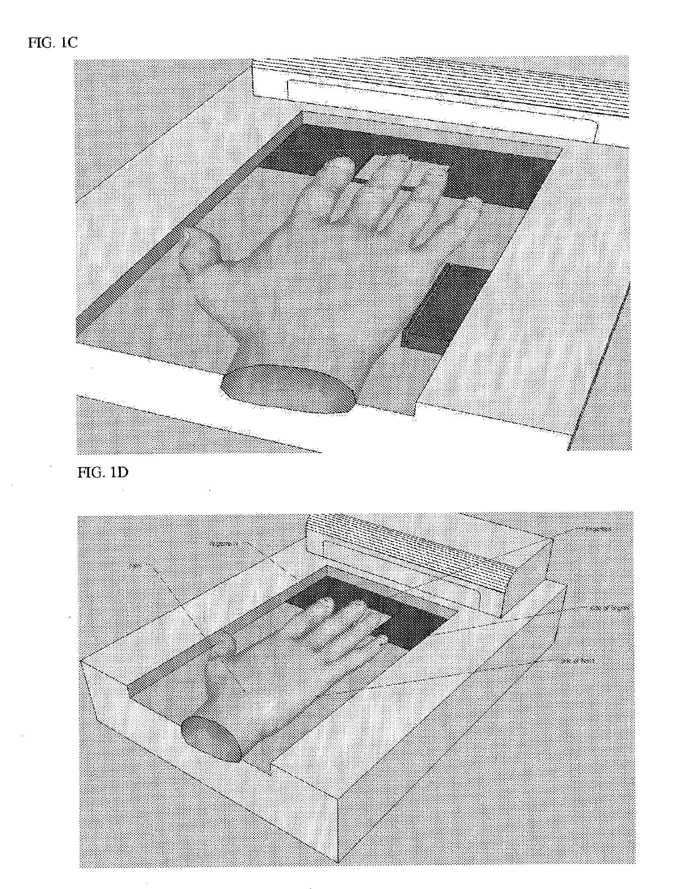

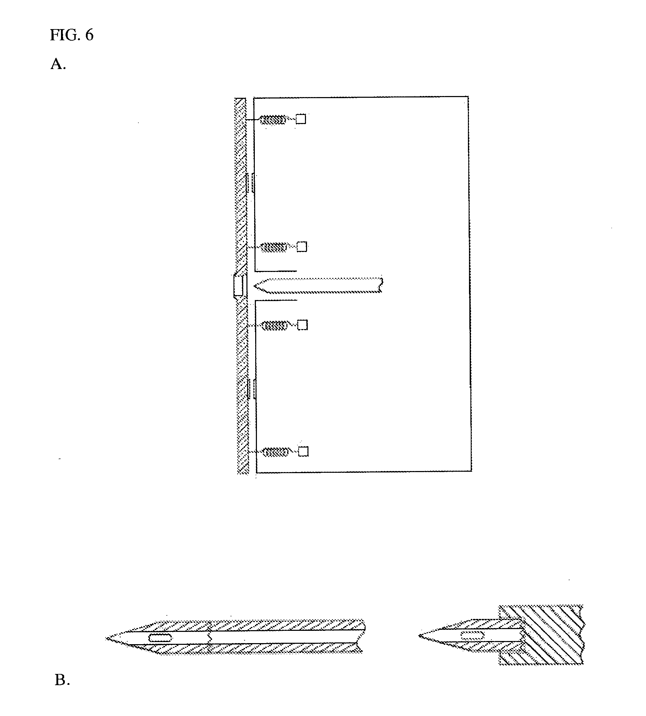

The presence sensor may be located in any suitable location relative to the platen surface that permits the sensor to detect that the appendage of the individual is placed upon to the platen to permit the collection of the at least one valley and ridge signature. The presence sensor also detects the position of the appendage relative to the EPD, such that, upon activation of the EPD, the EPD will contact the appendage, penetrate the epidermis and collect the biological sample of the individual, as shown in **FIG. 6A****.** In some embodiments, the presence sensor may be located below the plane of the platen surface, as shown in **FIG. 1** **B.** In other embodiments, the presence sensor may be located along a side of the platen, having an activating plate assembly oriented parallel to the length of a finger, toe, palm, sole, hand or foot (**FIG.1C** and **FIG. 7**). In these embodiments, the presence sensor elements may be directed perpendicularly to the length of a finger, toe, palm, sole, hand or foot in order to detect that the finger, toe, palm, sole, hand, or foot is positioned near the activating plate assembly in suitable proximity for the EPD to penetrate the epidermis once activated. In other embodiments, the presence sensor may be located at a side of the planar surface of the platen, at the top end of the imaging component surface, where the activating plate assembly is perpendicular to a distal end of a digit. In this distal end contacting arrangement, the presence sensor elements may be configured to detect when the distal end of the digit is positioned in suitable proximity for the EPD to penetrate the epidermis once activated.

Once the EPD penetrates the epidermis, a biological sample of the individual is collected by the EPD. Upon a further signal from the presence sensor or from other circuits of the imaging component or collection component, the EPD is retracted, withdrawing from penetration of the epidermis. Once the EPD is retracted, the biological sample may then be retrieved from the EPD for adding an identifier, archiving, further processing, and/or testing. In some embodiments, acquisition of the ridge and valley signature may be obtained concurrently with the collection of the biological sample. In other embodiments, the acquisition of the ridge and valley signature may be obtained before or after the collection of the biological sample. In some embodiments, when the epidermis penetrated by the EPD is located on a finger, including but not limited to a finger tip or a side of a finger, or a hand including but not limited to an ulnar side of the hand or a palm, then the at least one ridge and valley signature is obtained from at least one finger or the palm. In other embodiments, when the epidermis penetrated by the EPD is on a finger or an ulnar side of the hand, then the at least one ridge and valley signature is obtained from at least one finger. In yet other embodiments, when the epidermis is penetrated by the EPD is on a toe, including but not limited to a toe tip, a side of a toe, a foot, a fibular side of the foot, a sole of the foot, a heel of the foot, or a pad of the foot, then the at least one ridge and valley signature is obtained from the sole of the foot. In some embodiments, the at least one ridge and valley signature is obtained from the group consisting of at least one digit, a palm and a sole. In various embodiments, the epidermis penetrated by the EPD is on a digit, and the at least one ridge and valley signature is obtained from the digit. In other embodiments, the epidermis penetrated by the EPD is on a finger and the ridge and valley signature is obtained from a finger. In yet other embodiments, the epidermis penetrated by the EPD is on a hand and the ridge and valley signature is obtained from a finger.

After collection of the at least one ridge and valley signature, the signature, in analog or digital format, can be transmitted to a database having a plurality of ridge and valley signatures, as well as any other physical biometric data collected and deemed suitable to transmission. In some embodiments, the system includes a processor configured to transmit the ridge and valley signature obtained from the individual to at least one database which retains ridge and valley signatures of individuals. In some embodiments the database is selected from the group consisting of a forensic, criminal, identity, child identity, missing persons, immigration, department of motor vehicles, terrorist, paternity, arrestee, convict database or any combination thereof.

**Epidermal Puncturing Device.** In various embodiments, the biological collection component of the system includes an epidermal puncturing device (EPD) operationally connected to a presence sensor and configured to be activated to penetrate the epidermis of the individual and to retract, thereby extracting a biological sample which includes at least one nucleic acid. This device is a disposable sample acquisition device, and is designed to be easily replaceable in the biological sample collection component.

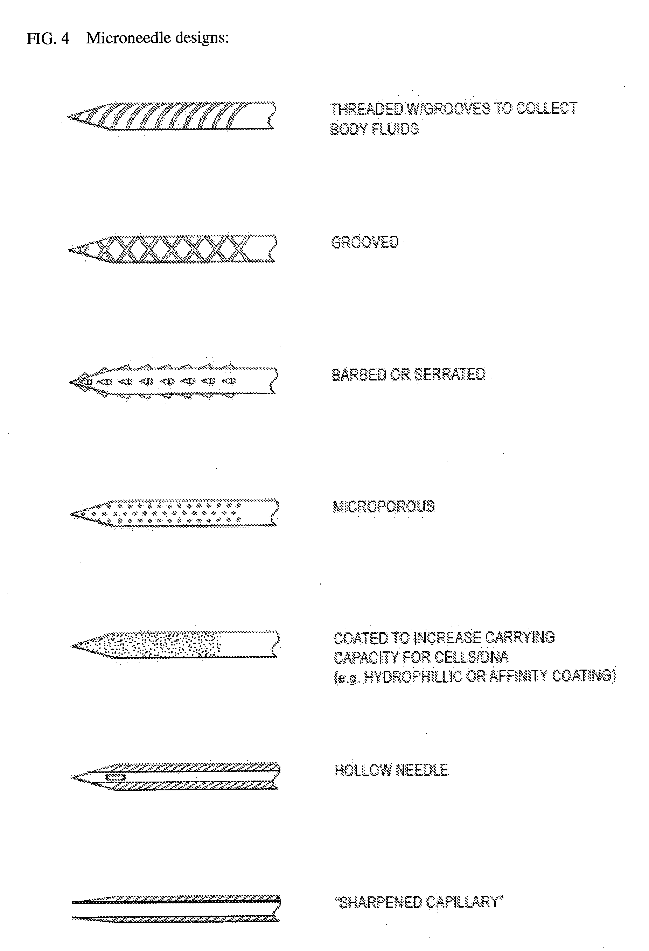

The nucleic acid sample is collected by a retractable epidermal puncturing device (EPD). The EPD has an anterior portion configured to penetrate the epidermis, which may be a needle, a microneedle, and a lancet, and are collectively referred to herein as "needle". The needle may have a sharp point. In some embodiments, the sharp point of the needle is beveled. In some embodiments, the anterior portion of the EPD is removable. **FIG. 4** illustrates possible needle designs and configurations of the needle. In some embodiments, the EPD may have an anterior portion having threaded grooves, grooves, barbs, serrations, microporous openings, or coatings. The EPD also has a posterior portion configured to be operationally connected to the presence sensor. The EPD may also have a lumen within the anterior or posterior portion of the EPD, or both. The EPD may be designed to collect the biological sample in a minimally invasive manner, and with minimal discomfort. Additionally the activation of the EPD by the presence sensor initiates the collection of the biological sample without requiring the individual to actively puncture himself/herself. This is an advantage over methods of biological sample collection which require the individual to actively puncture his/her own appendage. In some embodiments, collection of the biological sample into the EPD is assisted by applying vacuum to the EPD.

The EPD collects a biological sample which includes at least one nucleic acid from an individual by penetrating the epidermal layer of skin. **FIGS. 1A- 1B** illustrate two of many possible configurations/positions of an EPD within the system of the present teachings. **FIG. 1C** illustrates an individual's hand resting on a system of the present teachings with an EPD positioned at the ulnar side of the right hand. Additional sampling areas are illustrated for a hand in **FIG. 1** **D.** In various embodiments of the present teachings, an EPD can be positioned to obtain a biological sample from a sampling area of a finger, toe, hand or foot.

In some embodiments, before use, the EPD is resides below the platen surface, or within an activation assembly along a side of the platen so that a needle tip of the EPD does not protrude into the space above the platen. In some embodiments, the individual may initially position the appendage for imaging and sample collection without contacting the EPD.

When the EPD resides below the platen, there may be a hole in the platen to allow the EPD to move into activated and penetrating position. Once the presence sensor detects that an appendage is placed in proximity to the platen, the EPD is activated to move up through the hole in the platen surface to contact the appendage and penetrate the epidermis. In the activated position, the EPD now projects above the plane of the platen.

When the EPD resides within an activation assembly at the side of the platen, there is a hole in the face of the activation assembly through which the EPD moves to its activated and penetrating position. Once the presence sensor detects that an appendage is placed in proximity to the platen and to the activation assembly, the EPD is activated to move out of the activation assembly, contact the appendage, and penetrate the epidermis to collect the biological sample. In the activated position, the EPD now projects above the plane of the platen.

In some embodiments, "retractable" refers to movement of the EPD when it is activated or retracted by the presence sensor. In other embodiments, "retractable" refers to movement of the platen or activating assembly upon activation or retraction of the EPD to permit the EPD to contact the appendage and penetrate the epidermis.

In further embodiments of the present teachings, **FIG. 6A** illustrates a platen with one or more presence sensors for detecting the presence of an individual in conjunction with the system that scans the individual's finger, palm, toe or foot for the at least one valley and ridge signature. Upon sensing the appendage placed in proximity to the platen, the platen permits the EPD to activated and projected into the individual in a minimally invasive manner.

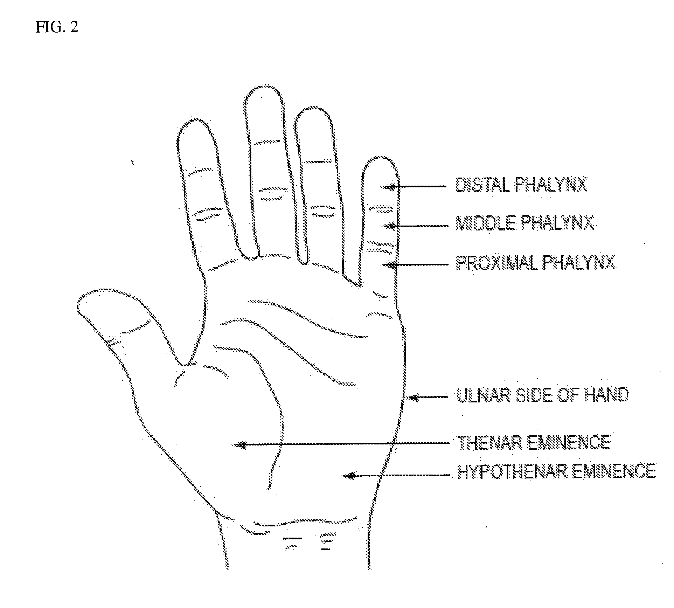

The system may configured to provide an EPD device to puncture the epidermis at a variety of positions where a biological sample can be obtained rapidly, easily and with minimum discomfort to the individual. Potential sites for obtaining a biological sample from an individual include a digit, including but not limited to a finger tip, a side of a finger, a toe tip, or a side of a toe; a hand, including but not limited to an ulnar side of the hand or a palm; a foot, including but not limited to a fibular side of the foot, a sole, a heel of the foot, or a pad of the foot. Some illustrative anatomical locations are shown in **FIG. 2** for the hand. References to anatomical structures, planes and surfaces follow the teaching of Grey's Anatomy: The Anatomical Basis of Clinical Practice, 40th edition (2008), Churchill-Livingstone, Elsevier, The hand has 5 digits, number 1 is the thumb, number 2 the index finger number 3 the middle finger, number 4 the ring finger and number 5 the little finger. In some embodiments, the palmar surface of the hand can be used as shown in **FIG. 2****.** The illustrated positions of **FIG. 2** can also apply to comparable areas of the plantar surface of the foot as would be understood by one of skill in the art.

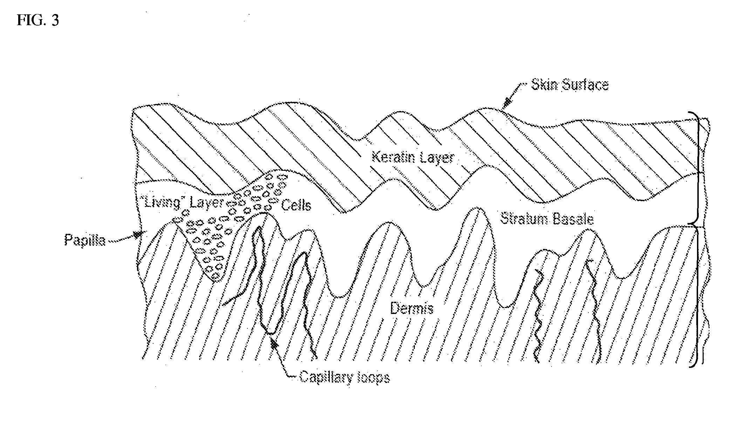

Below the epidermis layer of the skin is the dermis layer containing fibroblasts, macrophages and adipocytes, three cell types each having a nucleus containing nucleic acid. In addition the dermis has a vascular network of blood veins, arteries and lymph vessels containing white blood cells, having a nucleus, as does the erector pili muscle tissue, sebaceous glands and body fluids also present within the dermis. **FIG. 3** provides an illustration of some layers and components of the skin as seen in cross section. The lowest layer of the epidermis, the stratum basale, also contains living nucleated cells suitable for collection as part or whole of the biological sample. Additionally, sweat glands and vessels in the dermis may contain genetic materials in the form of intact cells or as free DNA.

In an embodiment of the present teachings the action of penetrating the skin with the needle portion of the EPD not only withdraws into a lumen of the needle biological fluid containing nucleated cells, but tissue and organ (skin) fragments, also containing nucleated cells may be captured on the outer surface of the needle by way of modifications to the needle's surface, addition of pores or projections. Capillary action or the application of a vacuum may assist in the collection of the biological sample as disclosed in published US Patent Application No. 2011/0172510, published July 14, 2011.

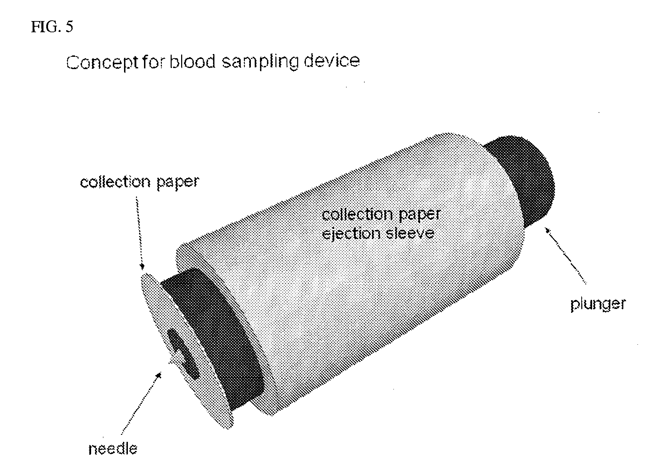

In other embodiments, when the EPD punctures the epidermis, the biological sample enters at least a portion of the EPD. In some embodiments, the biological sample enters the lumen of the needle. In further embodiments the needle lumen can contain a collection substrate or the needle can be surrounded by collection substrate located posterior to the point of the needle. The collection substrate may include absorbent material which may be composed of cotton, cellulose, filter paper and the like. The absorbent material may include or not include surface treatments such as ionic or non-ion materials capable of attracting and/or absorbing a biological sample. In some embodiments, the collection substrate may be filter paper, FTA® paper, Bode™ collection paper, a fibrous material and cotton. The collection substrate can be parallel, to the circumference of the needle, i.e., wrapped around the exterior or inserted within the lumen. Alternatively the collection substrate may be arranged in a disc-like fashion perpendicular to the exterior shaft of the needle. The collection substrate can come into direct contact with the biological sample or collect aspirates of biological material created during the puncturing and extraction processes. **FIG. 5** is an illustration of a biological sampling device, though scale and proportion should not be inferred from the drawing. **FIG. 6B** shows, in the illustration on the left, an EPD having a lumen and beveled needle tip at the end of its anterior portion. This embodiment also includes an imprinted fracture line to permit removal of the anterior portion, post-sample collection. The embodiment on the right side of the page shows an EPD having a posterior portion formed by a chuck, which permits ejection of the anterior portion of the EPD.

Following collection of the biological sample, retraction of the EPD, and extraction of the EPD from the biological sampling component, the anterior tip of the EPD can be removed. The anterior tip can contain biological sample alone or biological sample and absorbent material. Following collection, the needle tip plus biological sample alone or with collection substrate containing biological sample may be placed into a lysis buffer for extraction of the nucleic acid. Analysis for nucleic acid markers such as DNA markers for STRs, Indels, SNPs and combinations thereof may be performed as well as DNA sequencing methods. Reagents for analyzing nucleic acids are commercially available such as the AmpF/STR® Indentifiler® Direct PCR Amplification Kit (Applied Biosystems, Foster City, CA) and the PowerPlex® 18D System (Promega Corp. Madison, WI) in conjunction with Prep-n-Go™ Buffer (Applied Biosystems) following the manufacturer's instructions.

The EPD may be surrounded by a casing, and both casing and EPD may be maintained in a sterile condition, prior to collection of the biological sample. The EPD, and optionally, its casing may be configured to be removed from the imaging component after biological sample collection without contamination by an operator of the system. The EPD, and optionally, its casing, may be contained within a housing which is operationally connected to the presence sensor(s) which activate the EPD for sample collection.

In another embodiment of the present teachings, the system can further comprise at least a second imaging component for collecting a second image of the individual. The second image of the individual can be either the face of the individual or a component of the individual amenable to biometric identification. Suitable biometric images that may be collected as the second image include a retinal scan, or iris scan, the contours of the ear, facial recognition, hand geometry, foot geometry, voice, odor and scent. FIG. 7A illustrates a system capable of collecting the ridge and valley signature, biological sample and a facial image of the individual. **FIG. 7B** illustrates the EPD of **FIG. 7A****.**

**Identifier.** In still other embodiments of the present teachings, the system can further comprise an identifier for associating identifying information with the signature, biological sample, and, optionally a physical image, including any of a facial image, an iris image, a retinal image or an ear image of the individual. In some embodiments, the name of the individual is included in the identifier. The identifier may aid in correlating various collected samples and may preclude sample mix-up and human error as may occur with nonsystematic sample labeling for identifying collected data and samples. In various embodiments, the identifier is alphanumeric, graphical, magnetic, or electromagnetic. In some embodiments, the identifier is a barcode.

In yet other embodiments, the system additionally includes amplification, purification and separation components which may be used to: obtain the at least one nucleic acid from the biological sample; purify the at least one nucleic acid before further amplification and analysis; amplify the at least one nucleic acid; separate the amplified at least one nucleic acid; and detect the amplified at least one nucleic acid. Any or all of these additional components may be used to identify the individual.

**Kits.** The invention also provides for kits. A kit may include one or more of the EPD devices, and optionally instructions for use. A kit may further include reagents for the acquisition of the biological sample. The kit may further include reagents for archiving, shipment, or further processing of the biological sample. In some embodiments, further characterization of the biological sample is performed in a remote laboratory. Reagents and containers for shipping without contamination or degradation are included in a kit as provided by aspects of the invention.

**Methods.** The invention provides for methods of identifying an individual by collecting at least one nucleic acid sample and at least one ridge and valley signature of an individual. In various embodiments, the method includes providing an imaging component having a platen surface and a presence sensor wherein the platen surface is configured to permit an energy wave to penetrate the platen. The appendage, including and not limited to a digit, hand or foot of the individual is positioned in proximity to the platen and the presence sensor. When the presence sensor detects the contact of the appendage then the presence sensor activates an epidermal puncturing device (EPD) to contact the appendage and to penetrate the epidermis of the appendage. The activated EPD, upon penetrating the epidermis of the appendage, collects a biological sample including the at least one nucleic acid sample. While the individual's appendage is still engaged on the platen, the at least one ridge and valley signature is imaged by the energy wave. In some embodiments, the appendage is positioned on the platen, and optionally, pressed upon it. In some embodiments, the signature is collected electronically.

The method may additionally include conducting the biological sample for collection to a collection substrate positioned perpendicular to and surrounding the posterior portion of the EPD. The biological sample may alternatively be conducted to a collection substrate positioned within a lumen of the EPD. In yet another variation of the method, the biological sample is conducted to a lumen of the EPD. In some other embodiments of the method, vacuum may be applied to the EPD to collect the biological sample.

In some embodiments, the ridge and valley signature of the individual is transmitted to at least one database comprising ridge and valley signatures selected from the group consisting of a forensic, criminal, identity, child identity, missing persons, immigration, department of motor vehicles, terrorist, paternity, arrestee, convict database, access control, or any combination thereof.

In various embodiments, the biological sample of the individual is subjected to at least one of a polymerase chain reaction, a DNA sequencing reaction, STR analysis, SNP analysis, or indel analysis. In some embodiments, the biological sample is not purified further after collection, prior to use in any of the above reactions or analyses.

The method may additionally provide a step of collecting at least a second image of the individual, selected from a facial image, an iris image, a retinal image or an ear image of the individual.

The method may further include a step of providing an identifier configured to associate the biological sample of the individual with the at least one ridge and valley signature of the individual, wherein the identifier is alphanumeric, graphical, magnetic, or electromagnetic. In some embodiments, the identifier is a barcode. In some embodiments, the identifier also associates the at least a second image of the individual with the at least one ridge and valley signature and the biological sample of that individual. In various embodiments, the identifier further associates the biological sample, the at least one ridge and valley signature, and optionally the at least a second image with the name of the individual.

The steps of collecting the biological sample and collecting the at least one ridge and valley signature may be performed at the same time or may be performed sequentially, in any order. The imaging component may be an optical scanner or a capacitance scanner, wherein the energy wave is a light wave or an electrical wave. The biological sample may be a blood sample, a body fluid sample, or a tissue sample. The step of collecting the biological sample may be performed by penetrating the epidermis of a digit, including but not limited to a finger tip or a side of a finger; or a hand, including but not limited to an ulnar side of the hand or a palm, and the step of collecting at least one ridge and valley signature may be performed on at least one digit or the palm. In other embodiments, the step of collecting the biological sample may be performed by penetrating the epidermis of a toe, including but not limited to a toe tip or a side of a toe; or a foot, including but not limited to a fibular side of the foot, a sole of the foot, a heel of the foot, and a pad of the foot; and the step of collecting at least one ridge and valley signature may be performed on the sole of the foot.

Those having ordinary skill in the art will understand that many modifications, alternatives, and equivalents are possible. All such modifications, alternatives, and equivalents are intended to be encompassed herein.

### EXAMPLE 1

The following procedures are representative of procedures that can be employed for the collection, analysis and archiving/cataloging biological samples and biometric data from an individual.

### Example I

An individual's hand was cleaned with an alcohol swab followed by penetrating the epidermis using a 0.2mm diameter lancing device to a depth of 1.4mm. The tip of the needle was clipped off and placed into a well of a 96-well plate containing 2 uL of Prep-n-Go™ buffer. The plate was spun down and incubated at room temperature for about 10 minutes. 23 uL of Identifiler Direct master mix was added to the well containing the needle plus Prep-n-Go™ buffer and the mixture underwent polymerase chain reaction amplification (PCR) on an ABI9700 thermal cycler using standard conditions: 95C/11 m, then 30 cycles of 94C/20s, 59C/2m, 72C/1m followed by 60C/25 min and 4C - hold with the needle remaining in well during cycling. 1 uL PCR product was withdrawn following amplification and combined with 9uL HiDi formamide mixed with the LIZ®500 size standard. This mixture was injected into an ABI3500 capillary electrophoresis instrument with default Identifiler Direct analysis conditions; oven at 60C, prerun: 15kV, 180s, injection: 1.2kV, 24s, run: 15kV, 1210s, capillary length: 36cm and POP4™ separation polymer with G5 Dye set.

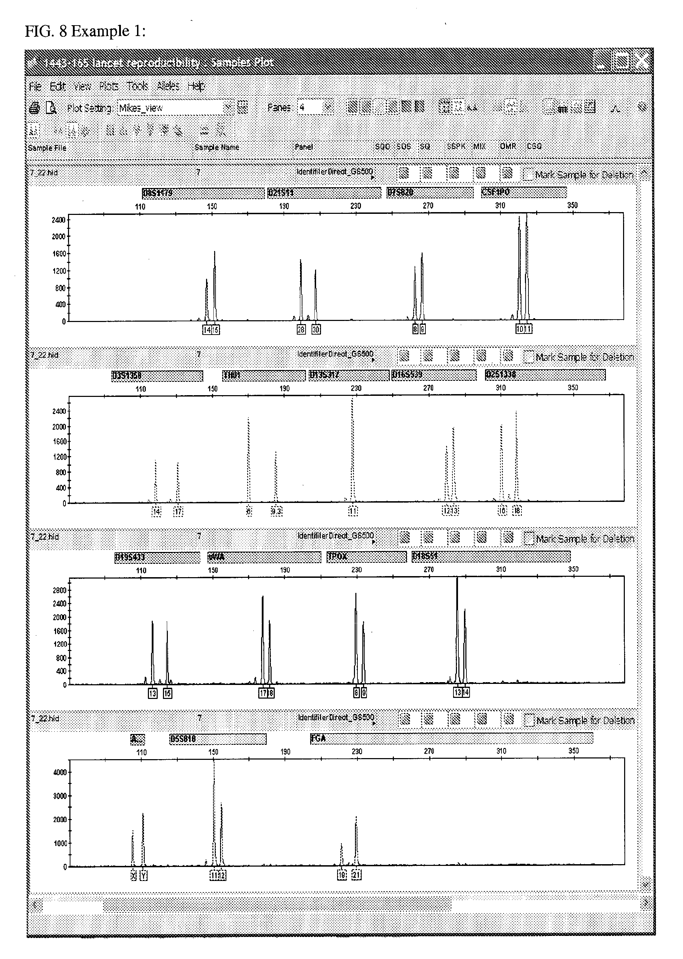

The resulting STR electropherogram was analyzed using GeneMapper® ID-X software (Applied Biosystems). **FIG. 8** shows the results of a direct amplification of the biological sample contained within the needle from the EPD. Full STR profiles for all 15 STR markers and the sex determination marker Amelogenin were obtained.

### Example II

An individual's left index finger was cleaned with an alcohol swab followed by penetrating the epidermis using a Micropoint Technologies hollow needle sampling device. The needle was a hollow pyramidal shaped, 1 mm length needle with a cotton absorbent material embedded behind the hollow needle. Following penetration of the epidermis, the cotton material was extracted from the needle with a sterile tweezers and cut into 3 pieces. Each of the 3 pieces was placed into a well of a 96-well plate containing 2 uL of Prep-n-Go™ buffer. And 23 uL of Identifiler Direct master mix was added to each well containing the cotton material plus Prep-n-Go™ buffer and the mixture underwent polymerase chain reaction amplification (PCR) on an ABI9700 thermalcycler using standard conditions: 95C/11 m, then 28 cycles of 94C/20s, 59C/2m, 72C/1 m followed by 60C/25 min and 4C - hold with the needle remains in well during cycling. 1 uL PCR product was withdrawn following amplification and combined with 9uL HiDi formamide mixed with the LIZ®500 size standard. This mixture was injected into an ABI3500 capillary electrophoresis instrument with default Identifiler Direct analysis conditions; oven at 60C, prerun: 15kV, 180s, injection: 1.2kV, 24s, run: 15kV, 1210s, capillary length: 36cm and POP4™ separation polymer with G5 Dye set.

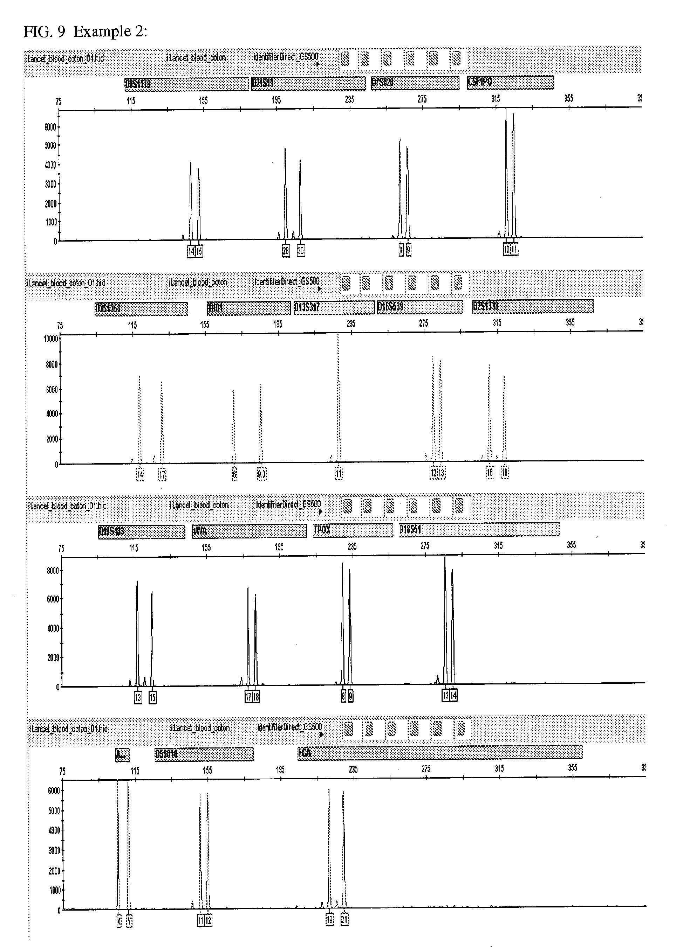

The resulting STR electropherogram was analyzed using GeneMapper® ID-X software (Applied Biosystems). **FIG. 9** shows the results of a direct amplification of the absorbent material contained within the needle from the EPD. Full STR profiles for all 15 STR markers and the sex determination marker Amelogenin were obtained.

While the foregoing specification teaches the principles of the present invention, with examples provided for the purpose of illustration, it will be appreciated by one skilled in the art from reading this disclosure that various changes in form and detail can be made without departing from the scope of the invention.

## Claims

1. A system for collecting a biological sample comprising at least one nucleic acid and at least one ridge and valley signature of an individual, the system comprising:
a) a biological collection component comprising an epidermal puncturing device operationally connected to a presence sensor, wherein the epidermal puncturing device is configured to be activated to penetrate the epidermis of the individual and to retract, thereby extracting the biological sample; and
b) at least a first imaging component configured to collect the at least one ridge and valley signature, wherein the at least first imaging component comprises i) a platen configured to receive an appendage of the individual, and ii) a presence sensor configured to a) detect when the appendage is placed in proximity to the platen, and b) activate the epidermal puncturing device to extract the biological sample.

2. The system of claim 1, wherein the presence sensor is a pressure sensor, optical sensor, electronic sensor, capacitive sensor or thermal sensor.

3. The system of any one of the preceding claims, wherein the presence sensor is located a) parallel to the length of a digit, palm, sole, hand or foot; b) perpendicular to the distal end of the digit; or c) resides below the platen, and wherein the presence sensor activates the epidermal puncturing device to a position above the plane of the platen thereby penetrating the epidermis of the subject.

4. The system of any one of the preceding claims, wherein the at least first imaging component is an optical scanner or a capacitance scanner.

5. The system of claim 4, wherein the optical scanner is an array of a plurality of light emitting diodes or a multispectral illuminator.

6. The system of any one of the preceding claims, further comprising a processor configured to transmit the ridge and valley signature of the individual to at least one database comprising ridge and valley signatures selected from the group consisting of a forensic, criminal, identity, child identity, missing persons, immigration, department of motor vehicles, terrorist, paternity, arrestee, convict database, access control and any combination thereof.

7. The system of any one of the preceding claims, further comprising an identifier configured to associate the biological sample of the individual with the at least one ridge and valley signature of the individual, wherein the identifier is alphanumeric, graphical, magnetic, or electromagnetic.

8. The system of claim 7, wherein the identifier is a barcode.

9. The system of any one of the preceding claims, wherein the epidermal puncturing device comprises:
a) an anterior portion configured to penetrate the epidermis, wherein the anterior portion is selected from the group of a needle, a microneedle, and a lancet; and
b) a posterior portion configured to be operationally connected to the presence sensor.

10. The system of claim 9, wherein the anterior portion of the epidermal puncturing device further comprises a beveled opening.

11. The system of any one of claims 9-10, wherein the anterior portion of the epidermal puncturing device further comprises a lumen.

12. The system of any one of claims 9-11, wherein the anterior portion of the epidermal puncturing device is removable.

13. The system of any one of claims 9-12, wherein the biological sample is conducted for collection to a) a collection substrate positioned perpendicular to and surrounding the posterior portion of the epidermal puncturing device; b) a collection substrate positioned within a lumen of the epidermal puncturing device; or c) a lumen of the epidermal puncturing device.

14. The system of claim 13, wherein the system is configured to apply vacuum to the epidermal puncturing device to assist in collecting the biological sample.

15. The system of any one of the preceding claims, wherein:
a) when the epidermis penetrated by the epidermal puncturing device is located on a finger or a hand, then the at least one ridge and valley signature is obtained from at least one finger or the palm; or
b) when the epidermis penetrated by the epidermal puncturing device is located on a toe or a foot, then the at least one ridge and valley signature is obtained from the sole of the foot.

16. The system of any one of the preceding claims, wherein the at least one ridge and valley signature is obtained from at least one finger.

17. A method of identifying an individual by collecting a biological sample comprising at least one nucleic acid sample and at least one ridge and valley signature of a individual, comprising the steps of:
a) providing an imaging component having a platen surface and a presence sensor wherein the platen surface is configured to permit an energy wave to penetrate the platen;
b) positioning an appendage of an individual in proximity to the platen and the presence sensor, wherein when the presence sensor detects the proximity of the appendage then the presence sensor activates an epidermal puncturing device to contact the appendage and to penetrate the epidermis of the appendage;
c) collecting the biological sample to the epidermal puncturing device; and
d) collecting the at least one ridge and valley signature imaged by the energy wave, wherein the signature is collected electronically.

18. The method of claim 17, wherein the ridge and valley signature is collected electronically.

19. The method of any one of claims 17-18, wherein the presence sensor detects the proximity of the appendage via pressure, an optical signal, an electronic signal, capacitance or temperature.

20. The method of any one of claims 17-19, wherein the steps of collecting the biological sample and collecting the at least one ridge and valley signature are performed at the same time.

21. The method of any one of claims 17-20, wherein the imaging component is an optical scanner or a capacitance scanner.

22. The method of any one of claims 17-21, wherein:
a) when the step of collecting the biological sample is performed by penetrating the epidermis of a finger or a hand, then the at least one ridge and valley signature is obtained from at least one finger or the palm; or
b) when the step of collecting the biological sample is performed by penetrating the epidermis of a toe or a foot, then the at least one ridge and valley signature is obtained from the sole of the foot.

23. The method of any one of claims 17-22, wherein the epidermal puncturing device comprises:
a) an anterior portion configured to penetrate the epidermis, wherein the anterior portion is selected from the group of a needle, a microneedle, and a lancet;
b) a posterior portion configured to be operationally connected to the presence sensor;
and,
optionally, wherein the anterior portion is removable.

24. The method of claim 23, further comprising the step of conducting the biological sample for collection to a) a collection substrate positioned perpendicular to and surrounding the posterior portion of the epidermal puncturing device; b) a collection substrate positioned within a lumen of the epidermal puncturing device; or c) a lumen of the epidermal puncturing device.

25. The method of claim 24, further comprising the step of applying vacuum to the epidermal puncturing device to collect the biological sample.

26. The method of any one of claims 17-25, further comprising the step of providing an identifier configured to associate the biological sample of the individual with the at least one ridge and valley signature of the individual, wherein the identifier is alphanumeric, graphical, magnetic, or electromagnetic.

27. The method of claim 26, wherein the identifier is a barcode.

28. The method of any one of claims 17-27, further comprising the step of transmitting the ridge and valley signature of the individual to at least one database comprising ridge and valley signatures selected from the group consisting of a forensic, criminal, identity, child identity, missing persons, immigration, department of motor vehicles, terrorist, paternity, arrestee, convict database, access control or any combination thereof.

29. The method of any one of claims 17-28, further comprising the step of subjecting the biological sample to at least one of a polymerase chain reaction, a DNA sequencing reaction, STR analysis, SNP analysis, or indel analysis.

30. The method of claim 29, wherein the at least one nucleic acid of the collected biological sample is not isolated or purified before use.

## Patentansprüche

1. System zum Sammeln einer biologischen Probe, umfassend mindestens eine Nukleinsäure und mindestens eine Erhebungs- und Rillensignatur eines Individuums, wobei das System umfasst:
a) eine biologische Sammelkomponente, umfassend eine epidermale Punktiervorrichtung, die betriebswirksam mit einem Präsenzmelder verbunden ist, wobei die epidermale Punktiervorrichtung konfiguriert ist, aktiviert zu werden, die Epidermis des Individuums zu durchdringen und daraus herauszuziehen, wodurch die biologische Probe entnommen wird; und
b) mindestens eine erste Abbildungskomponente, die zum Sammeln der mindestens einen Erhebungs- und Rillensignatur konfiguriert ist, wobei die mindestens eine Abbildungskomponente umfasst:
i) eine Platte, die zum Aufnehmen eines Anhangs des Individuums konfiguriert ist, und
ii) einen Präsenzmelder der zum a) Erkennen konfiguriert ist, wann der Anhang in Nähe der Platte angeordnet wird und b) die epidermale Punktiervorrichtung zum Extrahieren der biologischen Probe aktiviert.

2. System nach Anspruch 1, wobei der Präsenzmelder ein Drucksensor, ein optischer Sensor, ein elektronischer Sensor, ein kapazitiver Sensor oder ein Wärmesensor ist.

3. System nach einem der vorhergehenden Ansprüche, wobei der Präsenzmelder a) parallel zu der Länge eines Fingers oder einer Zehe, Hand- oder Fußfläche oder - sohle, Hand oder Fuß; b) senkrecht zu dem distalen Ende des Fingers oder der Zehe angeordnet ist; oder c) unterhalb der Platte liegt, und wobei der Präsenzmelder die epidermale Punktiervorrichtung in einer Position oberhalb der Ebene der Platte aktiviert, wodurch diese in die Epidermis des Subjekts eindringt.

4. System nach einem der vorhergehenden Ansprüche, wobei die mindestens erste Abbildungskomponente ein optischer Scanner oder ein Kapazitätsscanner ist.

5. System nach Anspruch 4, wobei der optische Scanner ein Array aus mehreren Leuchtdioden oder einer multispektralen Leuchte ist.

6. System nach einem der vorhergehenden Ansprüche, ferner umfassend einen Prozessor, der zum Übertragen der Erhebungs- und Rillensignatur des Individuums auf mindestens eine Datenbank konfiguriert ist, die Erhebungs- und Rillensignaturen umfasst, die ausgewählt sind aus der Gruppe, bestehend aus Datenbanken der Forensik, Kriminalistik, Identifizierung, Kinderidentifizierung, Vermissten, Einwanderer, Abteilung für motorbetriebene Fahrzeuge, Terroristen, Elternschaft, Inhaftierter, Verurteilter, Zugangssteuerung und jede beliebige Kombination davon.

7. System nach einem der vorhergehenden Ansprüche, ferner umfassend eine Kennung, die zum Verknüpfen der biologischen Probe des Individuums mit der mindestens einen Erhebungs- und Rillensignatur des Individuums konfiguriert ist, wobei die Kennung alphanumerisch, grafisch, magnetisch oder elektromagnetisch ist.

8. System nach Anspruch 7, wobei die Kennung ein Strichcode ist.

9. System nach einem der vorhergehenden Ansprüche, wobei die epidermale Punktiervorrichtung umfasst:
a) einen anterioren Abschnitt, der zum Eindringen in die Epidermis konfiguriert ist, wobei der anteriore Abschnitt ausgewählt ist aus der Gruppe, bestehend aus einer Nadel, einer Mikronadel und einer Lanzette; und
b) einen posterioren Abschnitt der zum betriebswirksamen Verbinden mit dem Präsenzmelder konfiguriert ist.

10. System nach Anspruch 9, wobei der anteriore Abschnitt der epidermalen Punktiervorrichtung ferner eine abgeschrägte Öffnung aufweist.

11. System nach einem der Ansprüche 9 bis 10, wobei der anteriore Abschnitt der epidermalen Punktiervorrichtung ferner ein Lumen aufweist.

12. System nach einem der Ansprüche 9 bis 11, wobei der anteriore Abschnitt der epidermalen Punktiervorrichtung entfernbar ist.

13. System nach einem der Ansprüche 9 bis 12, wobei die biologische Probe zum Sammeln auf a) ein Sammelsubstrat, das senkrecht zum und den posterioren Abschnitt der epidermalen Punktiervorrichtung umgebend angeordnet ist; b) ein Sammelsubstrat, das innerhalb eines Lumens der epidermalen Punktiervorrichtung angeordnet ist; oder c) ein Lumen der epidermalen Punktiervorrichtung geleitet wird.

14. System nach Anspruch 13, wobei das System konfiguriert ist, um Vakuum an die epidermale Punktiervorrichtung anzulegen, um das Sammeln der biologischen Probe zu unterstützen.

15. System nach einem der vorhergehenden Ansprüche, wobei:
a) wenn die Epidermis, die von der epidermalen Punktiervorrichtung durchdrungen wird, die eines Fingers oder einer Hand ist, die mindestens eine Erhebungs- und Rillensignatur von dem mindestens einen Finger oder der Handfläche erhalten wird; oder
b) wenn die Epidermis, die von der epidermalen Punktiervorrichtung durchdrungen wird, die einer Zehe oder eines Fußes ist, die mindestens eine Erhebungs- und Rillensignatur von der Fußsohle erhalten wird.

16. System nach einem der vorhergehenden Ansprüche, wobei die mindestens eine Erhebungs- und Rillensignatur von mindestens einem Finger erhalten wird.

17. Verfahren zum Identifizieren eines Individuums durch Sammeln einer biologischen Probe, umfassend mindestens eine Nukleinsäureprobe und mindestens eine Erhebungs- und Rillensignatur eines Individuums, umfassend die Schritte:
a) Bereitstellen einer Abbildungskomponente mit einer Plattenoberfläche und einem Präsenzmelder, wobei die Oberfläche der Platte so konfiguriert ist, dass eine Energiewelle die Platte durchdringen kann;
b) Positionieren eines Anhangs eines Individuums in Nähe der Platte und des Präsenzmelders, wobei, wenn der Präsenzmelder die Nähe des Anhangs erkennt, der Präsenzmelder eine epidermale Punktiervorrichtung zum Inkontakttreten mit dem Anhang und Durchdringen der Epidermis des Anhangs aktiviert;
c) Sammeln der biologischen Probe in der epidermalen Punktiervorrichtung; und
d) Sammeln der mindestens einen Erhebungs- und Rillensignatur, die von der Energiewelle abgebildet wird, wobei die Signatur elektronisch gesammelt wird.

18. Verfahren nach Anspruch 17, wobei die Erhebungs- und Rillensignatur elektronisch erfasst wird.

19. Verfahren nach einem der Ansprüche 17 bis 18, wobei der Präsenzmelder die Nähe des Anhangs über Druck, ein optisches Signal, ein elektronisches Signal, Kapazität oder Temperatur erkennt.

20. Verfahren nach einem der Ansprüche 17 bis 19, wobei die Schritte des Sammelns der biologischen Probe und des Sammelns der mindestens einen Erhebungs- und Rillensignatur gleichzeitig durchgeführt werden.

21. Verfahren nach einem der Ansprüche 17 bis 20, wobei die Abbildungskomponente ein optischer Scanner oder ein Kapazitätsscanner ist.

22. Verfahren nach einem der Ansprüche 17 bis 21, wobei:
a) wenn der Schritt des Sammelns der biologischen Probe durch Durchdringen der Epidermis eines Fingers oder einer Hand durchgeführt wird, die mindestens eine Erhebungs- und Rillensignatur von dem mindestens einen Finger oder der Handfläche erhalten wird; oder
b) wenn der Schritt des Sammelns der biologischen Probe durch Durchdringen der Epidermis einer Zehe oder eines Fußes durchgeführt wird, die mindestens eine Erhebungs- und Rillensignatur von der Fußsohle erhalten wird.

23. Verfahren nach einem der Ansprüche 17 bis 22, wobei die epidermale Punktiervorrichtung umfasst:
a) einen anterioren Abschnitt, der zum Eindringen in die Epidermis konfiguriert ist, wobei der anteriore Abschnitt ausgewählt ist aus der Gruppe, bestehend aus einer Nadel, einer Mikronadel und einer Lanzette;
b) einen posterioren Abschnitt, der zum betriebswirksamen Verbinden mit dem Präsenzmelder konfiguriert ist; und
wahlweise der anteriore Abschnitt entfernbar ist.

24. Verfahren nach Anspruch 23, ferner umfassend den Schritt des Leitens der biologischen Probe zum Sammeln auf a) ein Sammelsubstrat, das senkrecht zum und den posterioren Abschnitt der epidermalen Punktiervorrichtung umgebend angeordnet ist; b) ein Sammelsubstrat, das innerhalb eines Lumens der epidermalen Punktiervorrichtung angeordnet ist; oder c) in ein Lumen der epidermalen Punktiervorrichtung.

25. Verfahren nach Anspruch 24, ferner umfassend den Schritt des Anlegens eines Vakuums an die epidermale Punktiervorrichtung zum Sammeln der biologischen Probe.

26. Verfahren nach einem der Ansprüche 17 bis 25, ferner umfassend den Schritt des Bereitstellens einer Kennung, die zum Verknüpfen der biologischen Probe des Individuums mit der mindestens einen Erhebungs- und Rillensignatur des Individuums konfiguriert ist, wobei die Kennung alphanumerisch, grafisch, magnetisch oder elektromagnetisch ist.

27. Verfahren nach Anspruch 26, wobei die Kennung ein Strichcode ist.

28. Verfahren nach einem der Ansprüche 17 bis 27, ferner umfassend den Schritt des Übertragens der Erhebungs- und Rillensignatur des Individuums auf mindestens eine Datenbank, die Erhebungs- und Rillensignaturen umfasst, die ausgewählt sind aus der Gruppe, bestehend aus Datenbanken der Forensik, Kriminalistik, Identifizierung, Kinderidentifizierung, Vermissten, Einwanderer, Abteilung für motorbetriebene Fahrzeuge, Terroristen, Elternschaft, Inhaftierter, Verurteilter, Zugangssteuerung und jede beliebige Kombination davon.

29. Verfahren nach einem der Ansprüche 17 bis 28, ferner umfassend den Schritt des Aussetzens der biologischen Probe mindestens einer von Polymerase-Kettenreaktion, DNA-Sequenzierreaktion, STR-Analyse, SNP-Analyse oder Indel-Analyse.

30. Verfahren nach Anspruch 29, wobei die mindestens eine Nukleinsäure der gesammelten biologischen Probe vor der Verwendung nicht isoliert oder gereinigt wird.

## Revendications

1. Système de prélèvement d'un échantillon biologique comprenant au moins un acide nucléique et au moins une signature à crêtes et vallées d'un individu, le système comprenant :
a) un élément de prélèvement biologique comprenant un dispositif de perforation de l'épiderme connecté à un capteur de présence, le dispositif de perforation de l'épiderme étant configuré pour être activé afin de pénétrer dans l'épiderme de l'individu et de se rétracter de façon à extraire l'échantillon biologique, et
b) au moins un premier élément d'imagerie configuré pour prélever au moins une signature à crêtes et vallées, l'au moins un premier élément d'imagerie comprenant : i) une platine configurée pour recevoir un appendice de l'individu, et ii) un capteur de présence configuré pour a) détecter le moment où l'appendice est placé à proximité de la platine, et b) activer le dispositif de perforation de l'épiderme pour extraire l'échantillon biologique.

2. Système selon la revendication 1, dans lequel le capteur de présence est un capteur de pression, un capteur optique, un capteur électronique, un capteur capacitif ou un capteur thermique.

3. Système selon l'une quelconque des revendications précédentes, dans lequel le détecteur de présence est placé a) parallèlement à la longueur d'un doigt, d'une paume de main, d'une plante de pied, d'une main ou d'un pied ; b) perpendiculairement à l'extrémité distale du doigt ; ou c) est placé en dessous de la platine, et dans lequel le détecteur de présence active le dispositif de perforation de l'épiderme à une position au-dessus du plan de la platine de façon à pénétrer dans l'épiderme du sujet.

4. Système selon l'une quelconque des revendications précédentes, dans lequel l'au moins premier élément d'imagerie est un scanner optique ou un scanner capacitif.

5. Système selon la revendication 4, dans lequel le scanner optique est un réseau d'une pluralité de diodes électroluminescentes ou un illuminateur multi-spectral.

6. Système selon l'une quelconque des revendications précédentes, comprenant en outre un processeur configuré pour transmettre la signature à crêtes et vallées de l'individu à au moins une base de données comprenant des signatures à crêtes et vallées sélectionnées dans le groupe comprenant la médico-légale, la criminelle, l'identité, l'identité des enfants, les personnes disparues, l'immigration, le département des véhicules à moteur, le terrorisme, la paternité, les arrestations, la base de données des condamnés, le contrôle d'accès et toute combinaison de ceux-ci.

7. Système selon l'une quelconque des revendications précédentes, comprenant en outre un identificateur configuré pour associer l'échantillon biologique de l'individu à au moins une signature à crêtes et vallées de l'individu, l'identificateur étant alphanumérique, graphique, magnétique ou électromagnétique.

8. Système selon la revendication 7, dans lequel l'identificateur est un code à barres.

9. Système selon l'une quelconque des revendications précédentes, dans lequel le dispositif de perforation de l'épiderme comprend :
a) une partie antérieure configurée pour pénétrer dans l'épiderme, la partie antérieure étant choisie dans le groupe comprenant une aiguille, une micro-aiguille, et une lancette, et
b) une partie postérieure configurée pour être reliée fonctionnellement au détecteur de présence.

10. Système selon la revendication 9, dans lequel la partie antérieure du dispositif de perforation de l'épiderme comprend en outre un orifice en biseau.

11. Système selon l'une quelconque des revendications 9 à 10, dans lequel la partie antérieure du dispositif de perforation de l'épiderme comprend en outre une lumière.

12. Système selon l'une quelconque des revendications 9 à 11, dans lequel la partie antérieure du dispositif de perforation de l'épiderme est amovible.

13. Système selon l'une quelconque des revendications 9 à 12, dans lequel l'échantillon biologique est dirigé en vue du prélèvement vers a) un substrat de prélèvement positionné perpendiculairement à la partie postérieure du dispositif de perforation de l'épiderme et entourant celle-ci ; b) un substrat de prélèvement positionné à l'intérieur d'une lumière du dispositif de perforation de l'épiderme ; ou c) une lumière du dispositif de perforation de l'épiderme.

14. Système selon la revendication 13, dans lequel le système est configuré pour soumettre le dispositif de perforation de l'épiderme à une dépression afin de faciliter le prélèvement de l'échantillon biologique.

15. Système selon l'une quelconque des revendications précédentes, dans lequel :
a) lorsque l'épiderme, dans lequel le dispositif de perforation de l'épiderme a pénétré, est situé sur un doigt ou une main, alors au moins une signature à crêtes et vallées est obtenue à partir d'au moins un doigt ou une paume de main ; ou
b) lorsque l'épiderme, dans lequel le dispositif de perforation de l'épiderme a pénétré, est situé sur une orteil ou un pied, alors l'au moins une signature à crêtes et vallées est obtenue à partir de la plante du pied.

16. Système selon l'une quelconque des revendications précédentes, dans lequel l'au moins une signature à crêtes et vallées est obtenue à partir d'au moins un doigt.

17. Procédé d'identification d'un individu par prélèvement d'un échantillon biologique comportant au moins un échantillon d'acide nucléique et au moins une signature à crêtes et vallées d'un individu, comprenant les étapes consistant à :
a) produire un élément d'imagerie pourvu d'une surface de platine et d'un capteur de présence, la surface de platine étant configurée pour permettre à une onde énergétique de pénétrer dans la platine :
b) positionner un appendice d'un individu à proximité de la platine et du détecteur de présence, le capteur de présence activant un dispositif de perforation de l'épiderme, lorsque le détecteur de présence détecte la proximité de l'appendice, afin de venir en contact avec l'appendice et de pénétrer dans l'épiderme de l'appendice ;
c) prélever l'échantillon biologique pour l'amener au dispositif de perforation de l'épiderme ; et
d) prélever l'au moins une signature à crêtes et vallées par formation d'une image à l'aide de l'onde énergétique, la signature étant prélevée par des moyens électroniques.

18. Procédé selon la revendication 17, dans lequel la signature à crêtes et vallées est prélevée par des moyens électroniques.

19. Procédé selon l'une quelconque des revendications 17 à 18, dans lequel le détecteur de présence détecte la proximité de l'appendice par pression, signal optique, signal électronique, capacité ou température.

20. Procédé selon l'une quelconque des revendications 17 à 19, dans lequel les étapes consistant à prélever l'échantillon biologique et prélever l'au moins une signature à crêtes et vallées sont effectuées en même temps.

21. Procédé selon l'une quelconque des revendications 17 à 20, dans lequel l'élément d'imagerie est un scanner optique ou un scanner de capacité.

22. Procédé selon l'une quelconque des revendications 17 à 21, dans lequel :
a) lorsque l'étape de prélèvement de l'échantillon biologique est effectuée par pénétration dans l'épiderme d'un doigt ou d'une main, l'au moins une signature à crêtes et vallées est alors obtenue à partir d'au moins un doigt ou de la paume ; ou
b) lorsque l'étape de prélèvement de l'échantillon biologique est réalisée par pénétration dans l'épiderme d'un orteil ou d'un pied, l'au moins une signature à crêtes et vallées est alors obtenue à partir de la plante du pied.

23. Procédé selon l'une quelconque des revendications 17 à 22, dans lequel le dispositif de perforation de l'épiderme comprend :
a) une partie antérieure configurée pour pénétrer dans l'épiderme, la partie antérieure étant choisie dans le groupe comprenant une aiguille, une micro-aiguille, et une lancette ;
b) une partie postérieure configurée pour être reliée fonctionnellement au capteur de présence ; et
éventuellement, la partie antérieure étant amovible.

24. Procédé selon la revendication 23, comprenant en outre l'étape consistant à diriger l'échantillon biologique en vue du prélèvement vers a) un substrat de prélèvement positionné perpendiculairement à la partie postérieure du dispositif de perforation de l'épiderme et entourant celle-ci ; b) un substrat de prélèvement positionné à l'intérieur d'une lumière du dispositif de perforation de l'épiderme ; ou c) une lumière du dispositif de perforation de l'épiderme.

25. Procédé selon la revendication 24, comprenant en outre l'étape consistant à soumettre le dispositif de perforation de l'épiderme à une dépression pour prélever l'échantillon biologique.

26. Procédé selon l'une quelconque des revendications 17 à 25, comprenant en outre l'étape consistant à produire un identificateur configuré pour associer l'échantillon biologique de l'individu à l'au moins une signature à crêtes et vallées de l'individu, l'identificateur étant alphanumérique, graphique, magnétique ou électromagnétique.

27. Procédé selon la revendication 26, dans lequel l'identificateur est un code à barres.

28. Procédé selon l'une quelconque des revendications 17 à 27, comprenant en outre l'étape consistant à transmettre la signature à crêtes et vallées de l'individu à au moins une base de données comprenant des signatures à crêtes et vallées sélectionnées dans le groupe comprenant la médico-légale, la criminelle, l'identité, l'identité de l'enfant, les personnes disparues, l'immigration, le département des véhicules à moteur, le terrorisme, la paternité, les arrestations, les base de données des condamnés, le contrôle d'accès ou toute combinaison de ceux-ci.

29. Procédé selon l'une quelconque des revendications 17 à 28, comprenant en outre l'étape consistant à soumettre l'échantillon biologique à au moins une réaction de polymérisation en chaîne, une réaction de séquençage d'ADN, une analyse STR, une analyse SNP ou une analyse indel.

30. Procédé selon la revendication 29, dans lequel l'au moins un acide nucléique de l'échantillon biologique prélevé n'est pas isolé ou purifié avant son utilisation.
